# FASCICULE DE BREVET EUROPEEN

(11) **EP 0 486 353 B1**
(45) Date de publication et mention de la délivrance du brevet: **17.05.1995**
(21) Numéro de dépôt: 91402927.7
(22) Date de dépôt: 31.10.1991
(51) Int. Cl.: A61K 31/70, A61K 47/06

(54) **Composition anhydre à base de lactulose**
Lactulose enthaltende wasserfreie Zusammensetzung
Anhydrous composition containing lactulose

(30) Priorité: 02.11.1990 FR 9013619
(43) Date de publication de la demande: 20.05.1992
(73) Titulaire: Salsarulo, Odette, F-92100 Boulogne (FR)
(72) Inventeur: Salsarulo, Odette, F-92100 Boulogne (FR)
(74) Mandataire: Gutmann, Ernest

(56) Documents cités:
- FR-A- 2 154 322
- FR-A- 2 215 206
- J.E.R. REYNOLDS 'MARTINDALE - THE EXTRA PHARMACOPOEIA' 1989, THE PHARMACEUTICAL PRESS, LONDRES, GB LACTULOSE
- THE BRITISH JOURNAL OF CLINICAL PRACTICE Vol. 29, no. 9, 1975, pages 235 - 236; N. PORTER: 'THE USE OF LACTULOSE IN POST-HAEMORRHOIDECTOMY PATIENTS' '

## Description

La lactulose est un laxatif reconnu, agissant par son pouvoir osmotique au niveau de l'intestin.

Galéniquement, le lactulose se présente habituellement :
- soit sous forme de solution à 50%
- soit sous forme pure en poudre, mais il est, dans ce dernier cas, ingéré par les patients avec de l'eau.

Sous ces présentations, les doses orales actives de lactulose sont élevées, allant de 10 à 30 grammes/jour (pour un adulte) en valeur poudre pure (ce qui oblige à plusieurs prises dans la journée). L'administration par voie orale ne va pas sans problèmes relatifs à la tolérance du produit par l'organisme. Elle est susceptible d'entraîner des effets secondaires indésirables, tels que ballonnements ou flatulences.

L'invention a pour but de fournir une composition galénique permettant de remédier en grande partie à l'inconvénient sus-indiqué, c'est-à-dire de permettre l'administration quotidienne de doses plus faibles de lactulose, tout en conservant une activité laxative équivalente.

La composition ou forme galénique conforme à l'invention est caractérisée par l'incorporation de lactulose anhydre dans un véhicule également anhydre consistant en un mélange d'hydrocarbures paraffiniques purifiés, pharmaceutiquement acceptable par voie orale, ce véhicule ayant notamment un point de fusion entre 45°C et 60°C.

Il est à remarquer que les températures de fusion correspondant aux limites de l'intervalle de température sus-indiqué correspondent à des points de fusion capilaire mesurables avec une variabilité de ± 5% par la mise en oeuvre de la technique décrite dans la Pharmacopée Française, 10ème édition, 1983, V.611.

Ces nouvelles compositions pharmaceutiques à base de lactulose, complètement anhydres, non hydrophiles, peuvent être ingérées sans eau, donc comme telles par les patients. Ces compositions se sont avérées produire un effet osmotique laxatif maximum autorisant la réduction des doses de lactulose qui doivent être ingérées pour une activité laxative égale.

Le véhicule à base d'hydrocarbures est avantageusement constitué de distillats de carbures paraffiniques, affinés à un niveau leur assurant une complète innocuité. La composition de ces distillats est déterminée par la température de fusion retenue dans la fourchette sus-indiquée de points de fusion.

Une partie du véhicule pharmaceutique anhydre est constituée par de l'huile de paraffine (qualité Pharmacopée). Le véhicule préféré est donc constitué par un mélange de distillats de carbures paraffiniques contenant de l'huile de paraffine, la proportion de celle-ci variant de 1/3 à 2/3 du poids de la composition, selon la température de fusion choisie.

La proportion de lactulose par rapport à l'ensemble de la composition est avantageusement de l'ordre de 30 à 40% en poids de la composition totale.

L'utilisation de la composition selon l'invention, en lieu et place des compositions aqueuses antérieures, permet, à efficacité égale, une simplification de la posologie, en particulier la réduction, pratiquement de moitié, des doses efficaces de lactulose, au lieu des 10 à 30 grammes des préparations utilisées jusqu'à ce jour.

Il apparaît que les dosages quotidiens cliniquement utiles de lactulose administrés sous cette nouvelle formule sont de 5 à 10 grammes de lactulose par jour pour un adulte. Aux doses utilisées, la quantité d'huile de paraffine présente ne contribue que très modestement à l'effet laxatif de l'ensemble de la composition et n'explique pas à elle seule la réduction de la dose efficace de lalctulose.

Avantageusement, la composition est présentée sous une forme telle qu'elle permet l'administration de doses efficaces quotidiennes d'environ 5 grammes de lactulose par jour en une seule prise. A cette dose réduite la tolérance est fortement améliorée.

A titre d'exemple de proportions relatives aisément utilisables dans des compositions conformes à l'invention, on mentionnera celles contenant de l'ordre 5 grammes de lactulose pour de l'ordre de 10 grammes d'hydrocarbures paraffiniques pharmaceutiquement acceptables, soit l'équivalent d'une seule cuillèrée à soupe par jour de cette présentation galénique.

## Revendications

1. Composition ou forme galénique à base de lactulose caractérisée par l'association de lactulose anhydre avec un véhicule également anhydre consistant en un mélange d'hydrocarbures paraffiniques purifiés pharmaceutiquement acceptables.

2. Composition suivant la revendication 1, caractérisée en ce quel le véhicule est constitué d'un mélange de distillats de carbures paraffiniques affinés à un niveau leur assurant une complète innocuité et présentant un point de fusion entre 45°C et 60°C.

3. Composition selon la revendication 2, caractérisée par une proportion d'huile de paraffine, à raison de 1/3 à 2/3 du poids de la composition selon le point de fusion choisi.

4. Composition selon l'une quelconque des revendications 1 à 3, caractérisée en ce que la proportion de lactulose par rapport à l'ensemble de la composition est de l'ordre de 30 à 40% en poids de la composition totale.

5. Composition selon l'une quelconque des revendications 1 à 4, caractérisée par des doses permettant l'administration de lactulose réduites à raison de 5 à 10 grammes par jour.

6. Composition selon l'une quelconque des revendications 1 à 5, caractérisée en ce qu'elle contient de l'ordre de 5 grammes de lactulose pour environ 10 grammes d'hydrocarbures paraffiniques pharmaceutiquement acceptables.

7. Médicament laxatif anhydre, ingérable sans eau, pour l'administration orale, constitué par une composition selon l'une quelconque des revendications 1 à 6.

8. Procédé de production d'un médicament anhydre laxatif, à base de lactulose, caractérisé par l'association du lactulose sous une forme ingérable sans eau par les patients, avec un mélange d'hydrocarbures paraffiniques purifiés pharmaceutiquement acceptables, notamment tels que définis dans l'une quelconque des revendications 1 à 6 et, le cas échéant, dans les proportions en poids selon l'une quelconque des revendications 3 à 5.

## Claims

1. Lactulose-based galenical composition or form characterized by the combination of anhydrous lactulose with an equally anhydrous vehicle consisting of a mixture of pharmaceutically acceptable purified paraffinic hydrocarbons.

2. Composition according to Claim 1, characterized in that the vehicle is constituted of a mixture of paraffinic hydrocarbon distillates purified to an extent which ensures that they are completely harmless and exhibiting a melting point between 45°C and 60°C.

3. Composition according to Claim 2, characterized by a proportion of paraffin oil varying from 1/3 to 2/3 by weight of the composition depending on the melting point selected.

4. Composition according to any one of the Claims 1 to 3, characterized in that the proportion of the entire composition constituted by lactulose is about 30 to 40% by weight of the total composition.

5. Composition according to any one of the Claims 1 to 4, characterized by doses making possible the administration of lactulose at not more than 5 to 10 grams per day.

6. Composition according to any one of the Claims 1 to 5, characterized in that it contains about 5 grams of lactulose for about 10 grams of pharmaceutically acceptable paraffinic hydrocarbons.

7. Anhydrous laxative medicine, to be taken orally without water, constituted by a composition acording to any one of the Claims 1 to 6.

8. Process for the production of an anhydrous lactulose-based laxative medicine, characterized by the combination of lactulose in a form ingestible by patients without water with a mixture of pharmaceutically acceptable purified paraffinic hydrocarbons, in particular those defined in any one of the Claims 1 to 6 and, optionally, in the proportions by weight according to any one of the Claims 3 to 5.

## Patentansprüche

1. Zusammensetzung oder galenische Form auf Lactolose-Basis, gekennzeichnet durch die Vereinigung von wasserfreier Lactulose mit einem ebenfalls wasserfreien Träger, der aus einem Gemisch von Paraffinkohlenwasserstoffen, die in einem pharmazeutisch akzeptablen gereinigten Zustand sind, besteht.

2. Zusammensetzung nach Anspruch 1, dadurch gekennzeichnet, daß der Träger aus einem Gemisch von Paraffinkohlenwasserstoff-Destillaten besteht, die zu einem Grad raffiniert sind, der ihnen völlige Unschädlichkeit verleiht, und die einen Schmelzpunkt zwischen 45°C und 60°C aufweisen.

3. Zusammensetzung nach Anspruch 2, gekennzeichnet durch eine Menge an Paraffinöl von, je nach dem gewählten Schmelzpunkt, 1/3 bis 2/3 des Gewichts der Zusammensetzung.

4. Zusammensetzung nach einem der Ansprüche 1 bis 3, dadurch gekennzeichnet, daß die Lactulose-Menge in bezug auf die Gesamtheit der Zusammensetzung in der Größenordnung von 30 bis 40 Gew.-% der Gesamtzusammensetzung liegt.

5. Zusammensetzung nach einem der Ansprüche 1 bis 4, dadurch gekennzeichnet, daß sie die Verabreichung von Lactulose in reduzierten Dosen von 5 bis 10 g/Tag erlaubt.

6. Zusammensetzung nach einem der Ansprüche 1 bis 5, dadurch gekennzeichnet, daß sie Lactulose in der Größenordnung von etwa 5 g pro etwa 10 g pharmazeutisch akzeptabler Paraffinkohlenwasserstoffe enthält.

7. Wasserfreies, ohne Wasser einnehmbares Anführmittel zur oralen Verabreichung, das aus einer Zusammensetzung nach einen der Ansprüche 1 bis 6 besteht.

8. Verfahren zur Herstellung eines wasserfreien Anführmittels auf Lactulose-Basis, gekennzeichnet durch die Vereinigung von Lactulose, die in einer Form vorliegt, die von den Patienten ohne Wasser einnehmbar ist, mit einem Gemisch von Paraffinkohlenwasserstoffen, die in einem pharmazeutisch akzeptablen gereinigten Zustand sind, insbesondere so, wie in einem der Ansprüche 1 bis 6 definiert ist, und gegebenenfalls in den Gewichtsmengen gemäß einem der Ansprüche 3 bis 5.
